# EUROPEAN PATENT SPECIFICATION

(11) **EP 1 931 407 B1**
(45) Date of publication and mention of the grant of the patent: **24.07.2013**
(21) Application number: 06705038.5
(22) Date of filing: 20.03.2006
(51) Int. Cl.: A61M 5/50, A61M 5/32

(54) **SINGLE USE SAFETY SYRINGE HAVING A RETRACTABLE NEEDLE**
EINMAL-SICHERHEITSSPRITZE MIT ZURÜCKZIEHBARER NADEL
SERINGUE DE SÉCURITÉ JETABLE À AIGUILLE RÉTRACTABLE

(30) Priority: 06.09.2005 WO PCT/AU2005/001343
(43) Date of publication of application: 18.06.2008
(73) Proprietor: Global Medisafe Holdings Limited, Newcastle, NSW 2300 (AU)
(72) Inventor: WALTON, Graeme, Francis, Newcastle, NSW 2300 (AU); WALSH, Allan, Medowie, NSW 2318 (AU); LIN, Zuo, Qian, Zhejiang Provence (CN)
(74) Representative: Miller, James Lionel Woolverton
(86) International application number: PCT/AU2006/000372
(87) International publication number: WO 2007/028190

(56) References cited:
- EP-A1- 0 498 376
- EP-A1- 1 421 963
- CA-A1- 2 183 823
- CA-A1- 2 444 988
- CA-A1- 2 459 795
- US-A1- 2001 021 821
- US-A1- 2004 210 198
- US-A1- 2004 236 283
- US-A1- 2004 254 529
- US-A1- 2005 113 751

## Description

### Technical Field

This invention relates to a single use safety syringe. In particular it relates to a retractable needle for use in a single-use safety syringe, whereby the syringe user might be protected from injury by the needle after its use.

### Background

The danger of injury and possible infection from the HIV or hepatitis B virus to medical practitioners using needles in the normal course of their business is well documented.

Further, persons who are in the habit of administering drugs to themselves run a severe risk of contacting either of the specified viruses, or indeed contacting other viruses if a needle, once used, is reused in an unsterilised form.

There have been many proposals aimed at reducing the number of so-called needle-stick injuries and various attempts have been made to provide a safe system for disposal of such needles once used, but such prior proposals have had deficiencies.

US 2001/021821 and US 2004/236283 disclose prior art device according to the preamble of claim 1.

### Object of the Invention

It is therefore an object of the present invention to provide a single use safety syringe which employs a retractable surgical needle and for permanently storing that surgical needle, once used, in a substantially safe manner, so as to reduce needle stick injuries as well as preventing re-usage of the syringe thereby preventing cross infections of diseases.

It is a particular object of the invention to ameliorate against some or all of the known disadvantages of prior art syringes and to provide a simple safety syringe having a retractable needle which renders the syringe unable to be re-used. At the very least, the invention provides an alternate means for accommodating the needle of a used syringe to protect against accidental injury arising from unwanted contact with the exposed needle once the syringe has been used.

### Disclosure of the Invention

The invention in one broad form provides a single-use safety syringe having a retractable needle, the syringe including a hollow barrel, a plunger which slideably fits in the barrel, a piston which fits on the forepart of the plunger, and an actuator mechanism, which is located in the forepart of the barrel and which is able to be fitted therein through the open end of that forepart of the hollow barrel, the actuator mechanism having a stem for supporting a needle hub, and means located on the end of the plunger to engage the actuator and cause it to retract with the needle upon completion of the injection.

Where the actuator is located in the barrel, there is provided an inner flange on the barrel and a corresponding flange on the actuator mechanism, for fitment of the actuator from that open end of the barrel. The flanges are preferably shaped, for example having bevel surfaces on one side and right angled surfaces on the other, so that when the plunger is depressed (ie after the actuator has been thus fitted), the flange of the actuator mechanism locks against the corresponding inner flange of the barrel, for example by virtue of the opposing right angled surfaces coming into contact, so that in normal use the actuator will not be dislodged. Preferably a force of at least 98N may be endured. In this way, the actuator can not be dislodged from the barrel when the piston is depressed during an injection.

On the other hand the flanges need only withstand a lesser force than 98N in order to prevent the actuator from being withdrawn into the barrel when the needle of the syringe is inserted into the patient. Such force is preferably 49N. Indeed, in order that the actuator may be withdrawn into the barrel at the completion of the injection however, it is preferable that these opposing flange surfaces are bevelled so that only a lesser force is needed, ie a force preferably between 49N and 98N.

At the point of contact between the actuator and the barrel there is preferably provided at least one "O" type piston seal, sufficient that there is no leakage between the actuator and the barrel during use.

It will be appreciated that the actuator will be hollow so that fluid contained in the barrel may pass through it and hence through the needle which it supports. The end of the stem of the actuator is preferably provided with at least one flexible finger member. The plunger is preferably provided with a solid stem having a shaped head so that when the injection is completed, the head of the plunger stem enters into the hollow stem of the actuator and passes through the end thereof and is thereby engaged with the actuator, the one or more fingers retaining the head of the plunger stem beyond the stem of the actuator. When the plunger is pulled back, exerting a force preferably between 49 and 98N pull force, the one or more fingers can not break off, and the actuator will be successfully pulled into the barrel.

In order to prevent premature engagement of the head of the plunger stem with the fingers of the actuator stem, a depth stop is preferably provided to prevent the plunger from thus engaging with the actuator before retraction of the needle is required. Preferably such depth stop is provided in the form of a pin located in a hole located transverse the shaft of the plunger and located thereon so as to prevent the plunger coming into contact with the actuator until the pin is depressed, thereby avoiding early unwarranted destruction of the syringe.

The present invention is therefore a single use safety syringe which includes the following components: a hollow barrel, one plunger which fits the barrel and one piston which fits the forepart of the plunger. There is an actuator which fits the forepart of the barrel. Preferably on the actuator there is located a standard 6:100 luer taper. This taper is designed to receive the hub of a needle utilising interference fit. Alternatively, in order to provide a more positive interconnection between the actuator and the needle hub, a screw thread including so-called luer-lock thread may be employed with advantage.

Between the barrel and the outer actuator surface, there is fitted at least one seal. At the end of the barrel there is located at least one means for preventing complete depression of the plunger until the injection is complete and the syringe is to be rendered useless. At the forepart of the plunger there is located a pull back lock, preferably in the form of a head element for engagement with the forepart of the actuator's inner hole which is provided with flexible fingers, claws or the like, so that when the plunger is moved towards the actuator, the pull back hook in the forepart will enter into the actuator's engagement means easily. When the plunger is withdrawn, the actuator is pulled into the barrel, to realise the safe destruction thereof.

The safety syringe according to the preferred embodiments of the invention has the following advantages when compared with those of the existing market: firstly, it is provided with corresponding a flanges on the barrel and actuator to ensure that the actuator can not be dislodged and move back, an "O" type piston being provided between the actuator and the barrel, to make sure no liquid leaks out, and after injection, when the plunger is withdrawn the actuator can be pulled into the barrel easily, it can not be reused, achieving a truly safe syringe which is effectively destroyed; second, only need a minimal force to have the stem of the plunger enter into the flexible fingers o the stem of the actuator, thereby reducing the patient's pain which has been caused in injection in more recent times; third, the depth stop at the back-end of the plunger prevents the syringe being destroyed before it is used to give an injection through mis-operation causing premature destruction of the syringe.

Preferably the plunger is provided with a break point. Snapping off the plunger also renders the syringe both ineffective for subsequent use as well as providing additional safety in that there is no means by which the needle can be caused to re-emerge or extend from the syringe body.

Furthermore, it is preferable to provide means whereby the plunger can in fact be locked in position prior to being snapped off. This maybe achieved by utilising a sleeve or collar inserted in the end of the barrel, for example either by interference fit or by screwing it in place. A protrusion or lug may be provided on the plunger adjacent the break point and a passage for example like a thread may be provide in the sleeve or collar so that the plunger may be wound out through the collar or sleeve and locked by suitable means, eg flange or ratchet means so that when plunger is broken off at the break point there is no means by which it can be caused to re-enter the barrel as it is retained in the collar or sleeve.

### Brief Description of the Drawings

The invention may be better understood from the following non-limiting description of preferred embodiments, in which:
Figure 2 is an exploded view of a single-use safety syringe according to one embodiment of the invention;
Figure 3 is a cross-sectional view of the assembled syringe of Figure 1, showing the syringe prior to use with a cap covering and protecting the needle;
Figure 4 is a cross-sectional view of the syringe of Figure 2, showing the plunger drawn back, the barrel having been filled with injectable solution;
Figure 5 is a cross-sectional view of the syringe of the previous figures, at the completion of injection, the plunger having engaged the actuator;
Figure 6 is a cross-sectional view of the syringe of the previous figures, the plunger having been withdrawn so that the actuator and needle are safely withdrawn into the barrel of the syringe; and
Figure 7 is a cross section of the syringe of the earlier figures, the plunger having been locked in the sleeve at the end of the barrel and broken off at the break point rendering the syringe safely destroyed.

### Detailed Description of the Drawings

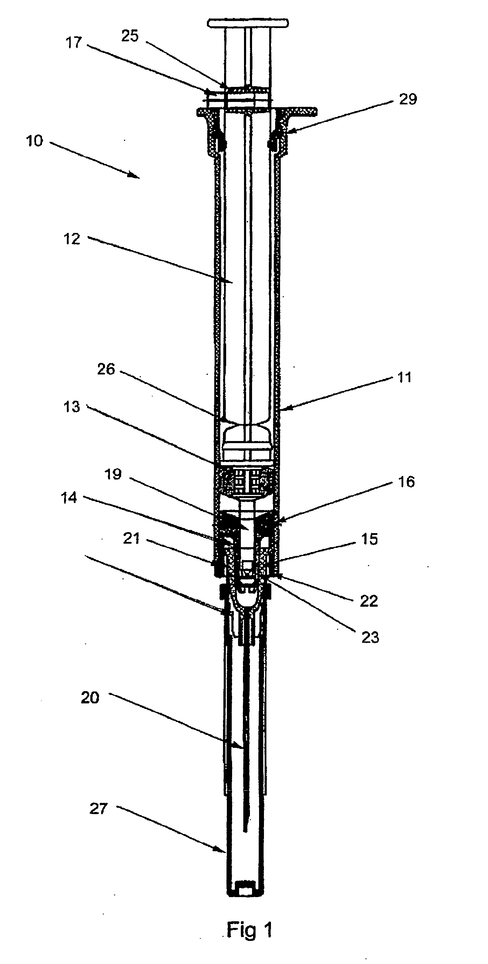

Referring to Figure 1 there is shown an exploded view of a syringe, generally referenced 10, according to a preferred embodiment of the invention.

The syringe includes a barrel 11, plunger 12, piston 13, actuator 14, needle hub 15 supporting a needle 20, "O" type piston 16, and depth stop 17. A cap 29 is provide to protect the needle prior to use.

An inner flange 21 is located in the foremost part of the barrel 11. A corresponding flange 22 is located on the actuator 14, for engagement of the actuator 14 when it is inserted into the barrel 11 from the open end of that foremost part of the barrel 11. The advantages of inserting the actuator 14 from that end of the barrel 11 lie in ease of assembly and less damage in situations where a needle hub 15 of a needle 20 is already fitted to the actuator 11, than would occur if the actuator were assembled by inserting it from the other (ie plunger) end of the syringe barrel 11.

Flexible spring fingers 23 are located on the end of the hollow stem 18 of the actuator 14. The solid stem 19 at the end of the plunger 13 is provided with a locking head 24. A hole 25 is located in the plunger 13 to accommodate the pin 7 which functions as a depth stop.

Break point 26 is provided on the plunger 13. The plunger also has flanges 28 which allow the plunger 13 to be twist locked in the twist lock sleeve 29 located in the end of the barrel 11.

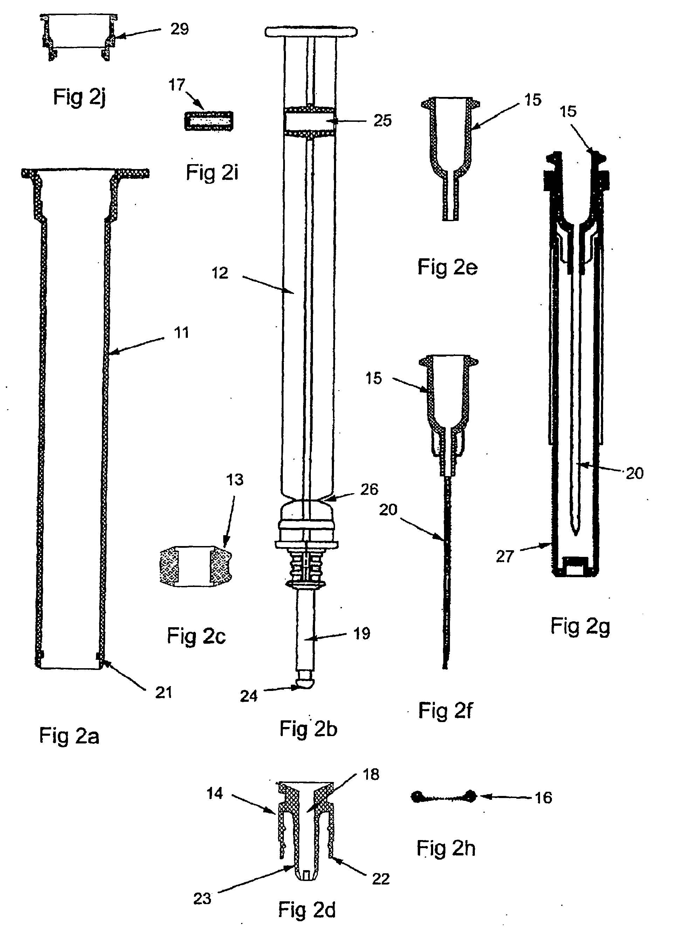

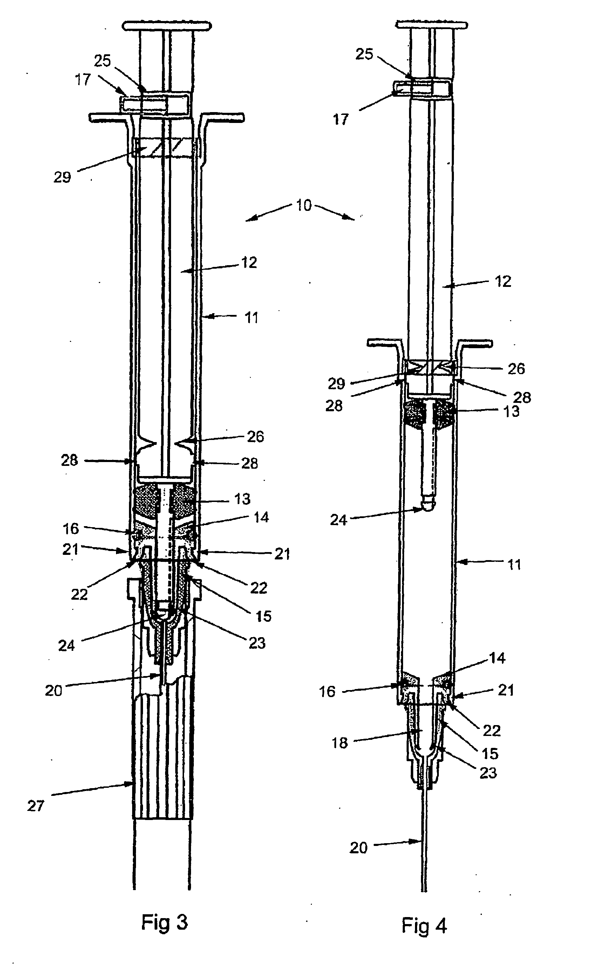

Referring generally to Figures 3 to 7, there is shown a syringe 10 which has the various parts illustrated as in Figure 2, the same reference numerals being utilised throughout.

The safety syringe 10 according to the invention possesses the following characters:

At the junction between the actuator 14 and barrel 11 there is located a sealing member 16 in the form of an "O" ring or the like. There is also located corresponding flanges 22, 21 on the actuator 14 and on the inner surface of the barrel 11 respectively. At each edge of the actuator flange 22 there is formed a bevel and a right angle respectively, the bevel and right angle correspond with a bevel and a right angle located on the flange 21 inside the barrel 11. In essence the orientation of the respective shapes of the two flanges 21, 22 means that in use, normal pressure as exerted during an injection will not cause the actuator to be dislodged outwardly or indeed inwardly. Indeed the use of the depth stop 17, prior to its being pressed in will ensure that this can't happen accidentally either. However, the use of the locking head 24 which engages the fingers 23 is in fact sufficient to easily draw the actuator 14 back, once the locking head 24 of the plunger 13 has been allowed to engage the actuator 14 by pressing in stop 17.

On the plunger 13 there is provided a break point 26, so that the plunger 13 can be broken off easily along the break point 26. At the back-end of plunger 13 there is located a depth stop 17 which can be pushed in, but which contacts the end of the barrel 11, until such time as it is pushed in so as to prevent the plunger 13 from being fully depressed and hence engaging with the actuator 14.

In Figure 3, the needle is protected by a cap 27 prior to use. The plunger 13 is prevented from engaging with actuator 14 by virtue of depth stop 17 which is in the out position.

In use the cap 27 is removed to reveal the needle 20 and the needle 20 is inserted into the fluid to be used for the injection (such as in a vial - not shown). The plunger 13 is then withdrawn as in Figure 4 to take fluid into the barrel 11.

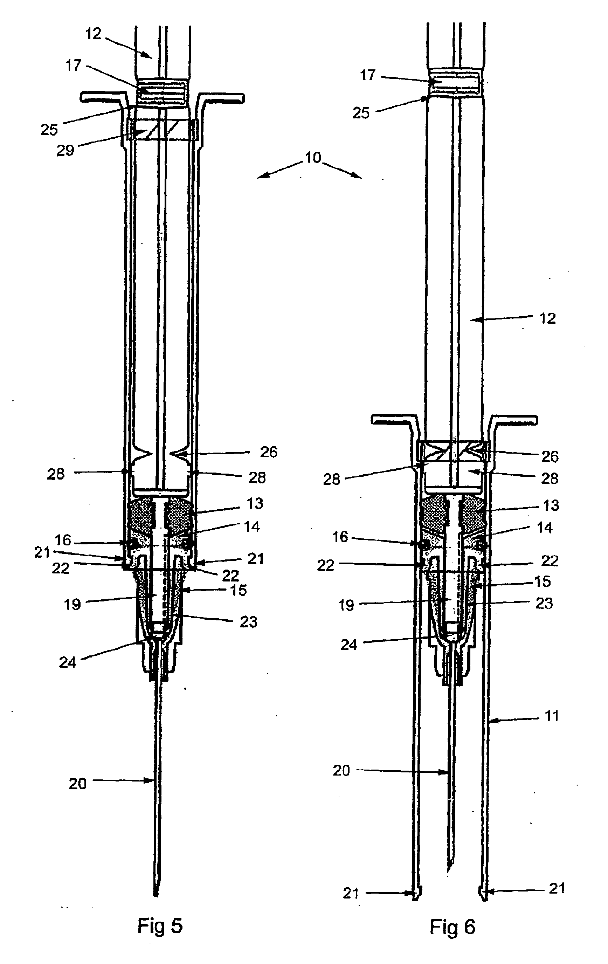

The injection is then performed after the depth stop 17 has been pushed in so that the plunger 13 now engages with the actuator 14 as in Figure 5.

At the end of the injection, the locking head 24 will have entered the hollow stem 18 of the actuator 14 and beyond into the flexible fingers 23 so that it is retained therein.

Upon withdrawing the plunger 13 once more, the actuator 14 and needle hub/needle assembly 15,20 are caused to be withdrawn into the barrel 11 of the syringe 10 as shown in Figure 5.

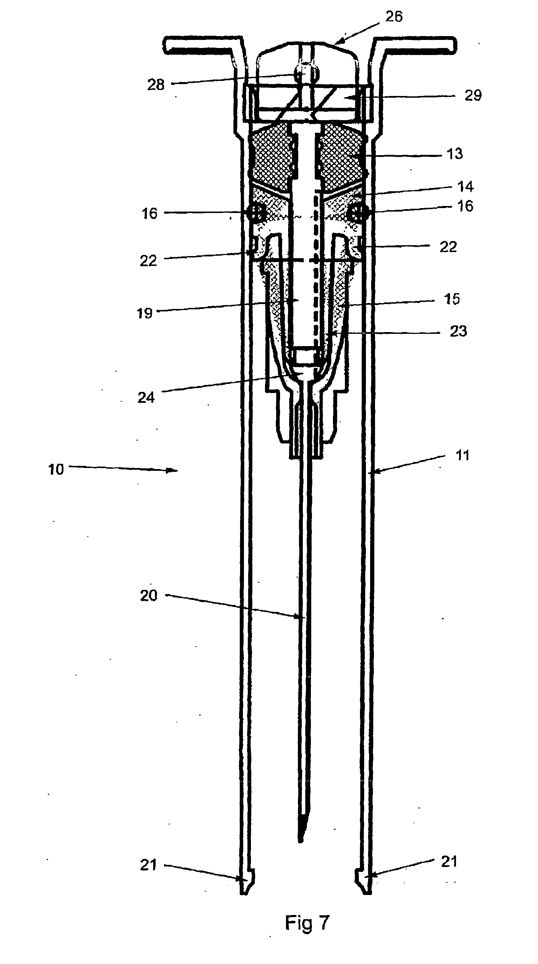

As shown in Figure 7, the flanges 28 located on the plunger 13 are twisted through the twist lock sleeve 29 fitted in the end of the barrel. Breaking off the plunger 13 at the break point 26 renders the syringe 10 totally safe and useless.

It will be appreciated by those skilled in the art that many modifications and variations may be made to the embodiments described herein without departing from the scope of the invention.

Throughout the specification the word "comprise" and its derivatives are intended to have an inclusive rather than exclusive meaning unless the context requires otherwise.

## Claims

1. A single use safety retractable syringe (10) having a retractable needle (20), the syringe including a hollow barrel (11), a plunger (13) which slideably fits in the barrel, a piston (13) which fits on the forepart of the plunger, and an actuator mechanism (14), which is located in the forepart of the barrel and which is able to be fitted therein through the open end of that forepart of the hollow barrel, the actuator mechanism having a stem (18) for supporting a needle hub (15), and means (24) located on the end of the plunger to engage the actuator and cause it to retract with the needle upon completion of the injection, **characterised in that** the stem (18) of the actuator is hollow and provided with at least one flexible finger (23) member and wherein in order to prevent premature engagement of the head (24) of the plunger stem with the fingers of the actuator stem, a depth stop (17) is provided to prevent the plunger from thus engaging with the actuator before retraction of the needle is required, the depth stop (17) being provided in the form of a pin located in a hole located transverse the shaft of the plunger and located thereon so as to prevent the plunger coming into contact with the actuator until the pin is depressed, thereby avoiding early unwarranted destruction of the syringe.

2. A single use safety retractable syringe according to claim 1, wherein at the point where the actuator is located in the barrel, there is provided an inner flange on the barrel and a corresponding flange on the actuator mechanism.

3. A single use safety retractable syringe according to claim 2, wherein the respective flanges are shaped, having bevel surfaces on one side and right angled surfaces on the other, so that when the plunger is depressed, the flange of the actuator mechanism locks against the corresponding inner flange of the barrel, by virtue of the opposing right angled surfaces coming into contact, so that in normal use the actuator will not be dislodged.

4. A single use safety retractable syringe according to claim 3 , wherein a force of at least 98N may be endured, so that the actuator can not be dislodged from the barrel when the piston is depressed during an injection under normal use conditions.

5. A single use safety retractable syringe according to either claim 3 or claim 4, wherein the flanges need only withstand a lesser force than 98N in order to prevent the actuator from being withdrawn into the barrel when the needle of the syringe is inserted into the patient.

6. A single use safety retractable syringe according to claim 5, wherein the lesser force is preferably 49N.

7. A single use safety retractable syringe according to any one of claims 5 to 6 wherein the opposing flange surfaces are bevelled so that only a lesser force is needed, ie a force preferably between 49N and 98N.

8. A single use safety retractable syringe according to any one of the preceding claims wherein at the point of contact between the actuator and the barrel there is provided at least one "O" type piston, sufficient that there is no leakage between the actuator and the barrel during use.

9. A single use safety retractable syringe according to any one of the preceding claims wherein the plunger is provided with a solid stem having a shaped head so that when the injection is completed, the head of the plunger stem enters into the hollow stem of the actuator and passes through the end thereof and is thereby engaged with the actuator, the one or more fingers retaining the head of the plunger stem beyond the stem of the actuator..

10. A single use safety retractable syringe according to claim 9, wherein when the plunger is pulled back, exerting a force between 49 and 98N pull force, the one or more fingers can not break off, and the actuator will be successfully pulled into the barrel.

11. A single use safety retractable syringe according to any one of the preceding claims wherein on the actuator there is located a standard 6:100 luer taper to receive the hub of a needle utilising interference fit.

12. A single use safety retractable syringe according to any one of claims 1 to 10, wherein in order to provide a more positive interconnection between the actuator and the needle hub, a screw thread including a so-called luer-lock thread is employed.

13. A single use safety retractable syringe according to any one of the preceding claims wherein the plunger is provided with a break point, so that the plunger is snapped off at completion of the injection rendering the syringe both ineffective for subsequent use as well as providing additional safety in that there is no means by which the needle can be caused to re-emerge or extend from the syringe body.

14. A single use safety retractable syringe according to claim 13 wherein means are provided whereby the plunger can be locked in position prior to being snapped off.

15. A single use safety retractable syringe according to claim 14 wherein a sleeve or collar is inserted in the end of the barrel by interference fit or by screwing it in place and one or more protrusions or lugs are provided on the plunger adjacent the break point and a passage like a thread is provided in the sleeve or collar so that the plunger may be twisted or wound out through the collar or sleeve and locked by suitable means, including flange or ratchet means so that when plunger is broken off at the break point there is no means by which it can be caused to enter the barrel again as it is retained in the collar or sleeve.

## Patentansprüche

1. Zurückziehbare Einweg-Sicherheitsspritze (10) mit einer zurückziehbaren Nadel (20), wobei die Spritze einen hohlen Zylinder (11) enthält; ein Druckstück (12), das gleitbar in den Zylinder passt; einen Kolben (13), der an den vorderen Teil des Druckstücks passt, und einen Betätigungsmechanismus (14), der sich im vorderen Teil des Zylinders befindet und durch das offene Ende dieses vorderen Teils des hohlen Zylinders darin eingepasst werden kann, wobei der Betätigungsmechanismus einen Schaft (18) für das Stützen eines Nadelansatzes (15) hat, und Mittel (24), die sich am Ende des Druckstücks befinden, um mit dem Betätigungsglied in Eingriff zu gehen und es bei der Beendigung der Injektion dazu zu bringen, sich mit der Nadel zurückzuziehen, **dadurch gekennzeichnet, dass** der Schaft (18) des Betätigungsglieds hohl und mit mindestens einem flexiblen Fingerglied (23) versehen ist, und wobei zum Verhindern eines frühzeitigen Eingriffs des Kopfstücks (24) des Schafts des Druckstücks mit den Fingern des Schafts des Betätigungsglieds ein Tiefenanschlag (17) vorgesehen ist, um zu vermeiden, dass das Druckstück auf diese Weise mit dem Betätigungsglied in Eingriff geht, bevor das Zurückziehen der Nadel erforderlich ist, wobei der Tiefenanschlag (17) in Form eines Stifts vorgesehen ist, der sich in einer quer zum Schaft des Druckstücks gelegenen Öffnung befindet, und der darauf derart positioniert ist, dass er das Druckstück daran hindert, mit dem Betätigungsglied in Kontakt zu kommen, bis der Stift zusammengedrückt wird, wodurch eine vorzeitige unnötige Zerstörung der Spritze verhindert wird.

2. Zurückziehbare Einweg-Sicherheitsspritze nach Anspruch 1, wobei an dem Punkt, an dem sich das Betätigungsglied im Zylinder befindet, ein innerer Flansch am Zylinder und ein entsprechender Flansch am Betätigungsmechanismus vorgesehen sind.

3. Zurückziehbare Einweg-Sicherheitsspritze nach Anspruch 2, wobei die jeweiligen Flansche derart geformt sind, dass sie auf einer Seite abgeschrägte Flächen und auf der anderen Seite rechtwinkelige Flächen haben, sodass beim Eindrücken des Druckstücks der Flansch des Betätigungsmechanismus den entsprechenden inneren Flansch des Zylinders sperrt, indem die gegenüber liegenden rechtwinkeligen Flächen in Kontakt geraten, sodass das Betätigungsglied beim normalen Gebrauch nicht hinausgedrückt wird.

4. Zurückziehbare Einweg-Sicherheitsspritze nach Anspruch 3, wobei einer Kraft von mindestens 98 N widerstanden werden kann, sodass das Betätigungsglied nicht aus dem Zylinder hinausgedrückt werden kann, wenn der Kolben bei einer Injektion unter normalen Gebrauchsbedingungen eingedrückt wird.

5. Zurückziehbare Einweg-Sicherheitsspritze nach Anspruch 3 oder Anspruch 4, wobei die Flansche nur einer geringeren Kraft als 98 N widerstehen müssen, um zu verhindern, dass das Betätigungsglied in den Zylinder zurückgezogen wird, wenn die Nadel der Spritze in den Patienten eingeführt wird.

6. Zurückziehbare Einweg-Sicherheitsspritze nach Anspruch 5, wobei die geringere Kraft vorzugsweise 49 N beträgt.

7. Zurückziehbare Einweg-Sicherheitsspritze nach einem der Ansprüche 5 bis 6, wobei die gegenüber liegenden Flächen der Flansche abgeschrägt sind, sodass nur eine geringere Kraft erforderlich ist, d. h. vorzugsweise eine Kraft zwischen 49 N und 98 N.

8. Zurückziehbare Einweg-Sicherheitsspritze nach einem der vorhergehenden Ansprüche, wobei am Kontaktpunkt des Betätigungsglieds und des Zylinders mindestens ein Kolben vom "O"-Typ vorgesehen ist, was dafür ausreichend ist, dass beim Gebrauch keine Undichtigkeiten zwischen dem Betätigungsglied und dem Zylinder auftreten.

9. Zurückziehbare Einweg-Sicherheitsspritze nach einem der vorhergehenden Ansprüche, wobei das Druckstück mit einem soliden Schaft mit einem geformten Kopfstück versehen ist, sodass das Kopfstück des Schafts des Druckstücks beim Beendigen der Injektion in den hohlen Schaft des Betätigungsglieds eintritt und sich durch das Ende davon bewegt, wodurch es mit dem Betätigungsglied in Eingriff geht und der eine oder die mehreren Finger das Kopfstück des Schafts des Druckstücks unter dem Schaft des Betätigungsglieds halten.

10. Zurückziehbare Einweg-Sicherheitsspritze nach Anspruch 9, wobei beim Zurückziehen des Druckstücks, wobei eine Zugkraft zwischen 49 und 98 N ausgeübt wird, der eine oder die mehreren Finger nicht abbrechen können und das Betätigungsglied erfolgreich in den Zylinder gezogen wird.

11. Zurückziehbare Einweg-Sicherheitsspritze nach einem der vorhergehenden Ansprüche, wobei sich am Betätigungsglied ein standardmäßiger 6/100-Luer-Konus befindet, um den Ansatz einer Nadel mittels Presspassung aufzunehmen.

12. Zurückziehbare Einweg-Sicherheitsspritze nach einem der Ansprüche 1 bis 10, wobei zum Bereitstellen einer positiveren Verbindung zwischen dem Betätigungsglied und dem Nadelansatz ein Schraubgewinde eingesetzt wird, einschließlich eines sogenannten Luer-Lock-Gewindes.

13. Zurückziehbare Einweg-Sicherheitsspritze nach einem der vorhergehenden Ansprüche, wobei das Druckstück mit einer Bruchstelle versehen ist, sodass das Druckstück bei der Beendigung der Injektion abgebrochen wird, wodurch sowohl die Spritze für nachfolgenden Gebrauch unbrauchbar gemacht als auch zusätzliche Sicherheit bereitgestellt wird, indem es kein Mittel gibt, durch das die Nadel dazu gebracht werden kann, aus dem Körper der Spritze wieder herauszuragen oder sich davon zu erstrecken.

14. Zurückziehbare Einweg-Sicherheitsspritze nach Anspruch 13, wobei Mittel vorgesehen sind, durch die das Druckstück an seiner Position arretiert werden kann, bevor es abgebrochen wird.

15. Zurückziehbare Einweg-Sicherheitsspritze nach Anspruch 14, wobei in das Ende des Zylinders eine Hülse oder ein Kragen durch Presspassung eingesetzt oder in Position festgeschraubt ist und neben der Bruchstelle ein oder mehrere Vorsprünge oder Ansätze vorgesehen sind und eine Passage, z. B. ein Gewinde, in der Hülse oder dem Kragen vorgesehen ist, sodass das Druckstück durch den Kragen oder die Hülse herausgedreht oder herausgewunden und durch geeignete Mittel gesperrt werden kann, einschließlich Flansch- oder Einrast-Mittel, sodass es nach dem Abbrechen des Druckstücks an der Bruchstelle kein Mittel gibt, durch das es dazu gebracht werden kann, wieder in den Zylinder einzutreten, da es durch den Kragen oder die Hülse zurückgehalten wird.

## Revendications

1. Seringue de sécurité rétractable jetable (10) comprenant une aiguille rétractable (20), ladite seringue comprenant un tube creux (11), un plongeur (13) introduit par ajustement glissant dans le tube, un piston (13) monté sur la partie avant du plongeur, et un mécanisme actionneur (14) situé dans la partie avant du tube et pouvant être monté dans celui-ci par l'extrémité ouverture de ladite partie avant du tube creux, ledit mécanisme actionneur comprenant une tige (18) pour supporter un pavillon d'aiguille (15) et des moyens (24) disposés à l'extrémité du plongeur pour entrer en contact avec l'actionneur et provoquer sa rétraction avec l'aiguille quand l'injection est achevée, **caractérisée en ce que** la tige (18) de l'actionneur est creuse et dotée d'au moins un élément formant griffe (23) souple et dans laquelle, pour empêcher le contact prématuré de la tête (24) de la tige du plongeur avec les griffes de la tige d'actionneur, une butée de profondeur (17) est prévue de façon à empêcher la mise en prise du plongeur avec l'actionneur avant que la rétraction de l'aiguille soit requise, la butée de profondeur (17) étant conçue en forme de broche disposée dans un orifice ménagé dans un sens transversal dans l'arbre du plongeur afin d'éviter la mise en contact du plongeur avec l'actionneur tant que la broche n'est pas poussée vers l'intérieur, de façon à éviter la destruction prématurée et injustifiée de la seringue.

2. Seringue de sécurité rétractable jetable selon la revendication 1, dans laquelle à l'endroit où l'actionneur est disposé dans le tube, une bride interne est disposée sur le tube et une bride correspondante est disposée sur le mécanisme actionneur.

3. Seringue de sécurité rétractable jetable selon la revendication 2, dans laquelle les brides respectives sont façonnées de façon à présenter des surfaces biseautées sur un côté et des surfaces en angle droit sur l'autre, de manière à ce que la bride du mécanisme actionneur se bloque contre la bride interne correspondante du tube quand le plongeur est poussé, par la mise en contact des surfaces en angle droit opposées, de sorte que l'actionneur ne soit pas délogé lors d'une utilisation normale.

4. Seringue de sécurité rétractable jetable selon la revendication 3, dans laquelle une force d'au moins 98 N peut être supportée, de sorte que l'actionneur ne puisse pas être délogé du tube quand le piston est poussé au cours d'une injection dans des conditions d'utilisation normales.

5. Seringue de sécurité rétractable jetable selon la revendication 3 ou 4, dans laquelle les brides ne nécessitent de supporter qu'une force inférieure à 98 N afin d'empêcher l'actionneur d'être rétracté dans le tube quand l'aiguille de la seringue est insérée dans le corps du patient.

6. Seringue de sécurité rétractable jetable selon la revendication 5, dans laquelle la force inférieure est de préférence de 49 N.

7. Seringue de sécurité rétractable jetable selon l'une quelconque des revendications 5 ou 6, dans laquelle les surfaces formant brides opposées sont biseautées de façon à ce qu'une force inférieure soit nécessaire, c'est-à-dire une force allant de préférence de 49 à 98 N.

8. Seringue de sécurité rétractable jetable selon l'une quelconque des revendications précédentes, dans laquelle au moins un piston de type « O » est disposé au point de contact entre l'actionneur et le tube, ledit piston étant apte à empêcher les fuites entre l'actionneur et le tube durant l'utilisation.

9. Seringue de sécurité rétractable jetable selon l'une quelconque des revendications précédentes, dans laquelle le plongeur est doté d'une tige pleine dotée d'une tête façonnée de telle façon que quand l'injection est achevée, la tête de la tige de plongeur pénètre dans la tige creuse de l'actionneur et traverse l'extrémité de celui-ci de façon à entrer en prise avec l'actionneur, la ou les griffe(s) retenant la tête de la tige de plongeur en dessous de la tige de l'actionneur.

10. Seringue de sécurité rétractable jetable selon la revendication 9, dans laquelle quand le plongeur est tiré vers l'arrière sous une force de traction allant de 49 à 98 N, la ou les griffe(s) ne peut (peuvent) pas se rompre et l'actionneur est tiré efficacement à l'intérieur du tube.

11. Seringue de sécurité rétractable jetable selon l'une quelconque des revendications précédentes, dans laquelle un embout Luer 6 : 100 standard est disposé sur l'actionneur pour recevoir le pavillon d'une aiguille par ajustement avec serrage.

12. Seringue de sécurité rétractable jetable selon l'une quelconque des revendications 1 à 10, dans laquelle pour assurer une interconnexion plus sûre entre l'actionneur et le pavillon d'aiguille, un filetage comprenant un filetage du type Luer-Lock est utilisé.

13. Seringue de sécurité rétractable jetable selon l'une quelconque des revendications précédentes, dans laquelle le plongeur est doté d'un point de rupture, de sorte que le plongeur se casse quand l'injection est achevée, de façon à rendre la seringue inutilisable une seconde fois tout en procurant une sécurité supplémentaire du fait que l'aiguille ne peut pas émerger de nouveau ou s'étendre hors du corps de seringue.

14. Seringue de sécurité rétractable jetable selon la revendication 13, dans laquelle des moyens sont fournis permettant de bloquer le plongeur en place avant qu'il soit cassé.

15. Seringue de sécurité rétractable jetable selon la revendication 14, dans laquelle un manchon ou collier est inséré à l'extrémité du tube par ajustement avec serrage ou par filetage et une ou plusieurs saillies ou oreilles est/sont disposées sur le plongeur à proximité du point de rupture et un passage de type fileté est ménagé dans le manchon ou collier de façon à ce que le plongeur puisse être dévissé hors du manchon ou collier et bloqué par des moyens appropriés comprenant des moyens à bride ou à cliquet, de telle façon que lorsque le plongeur est rompu au niveau du point de rupture il ne peut d'aucune manière être réintroduit dans le tube et il est retenu dans le collier ou le manchon.
